# EUROPÄISCHE PATENTSCHRIFT

(11) **EP 2 634 578 B1**
(45) Veröffentlichungstag und Bekanntmachung des Hinweises auf die Patenterteilung: **01.04.2015**
(21) Anmeldenummer: 13156499.9
(22) Anmeldetag: 25.02.2013
(51) Int. Cl.: G01N 33/68, G01N 33/86, G01N 33/53, G01N 33/543

(54) **Verfahren zur Erkennung von Interferenzen in in vitro-diagnostischen Testen**
Method for detecting interference in in-vitro diagnostic tests
Procédé de détection d'interférences dans des tests de diagnostic in vitro

(30) Priorität: 29.02.2012 EP 12157414
(43) Veröffentlichungstag der Anmeldung: 04.09.2013
(73) Patentinhaber: Siemens Healthcare Diagnostics Products GmbH, 35041 Marburg (DE)
(72) Erfinder: Patzke, Juergen, 35043 Marburg (DE)

(56) Entgegenhaltungen:
- BOSCATO L M ET AL: "INCIDENCE AND SPECIFICITY OF INTERFERENCE IN TWO-SITE IMMUNOASSAYS", CLINICAL CHEMISTRY, AMERICAN ASSOCIATION FOR CLINICAL CHEMISTRY, WASHINGTON, DC, Bd. 32, Nr. 8, 1. Januar 1986 (1986-01-01) , Seiten 1491-1495, XP002918651, ISSN: 0009-9147
- Erich Kunzmann: "Assay-Prinzip Ristocetin-Kofaktor BC von Willebrand Reagenz (vWF:RCo)", , 13. Januar 2012 (2012-01-13), XP055027877, Gefunden im Internet: URL:http://www.klinikum.uni-heidelberg.de/ fileadmin/zentrallabor/PDF/Prinzip_vWF-Akt ivitaet.pdf [gefunden am 2012-05-23]
- Unknown: "HemosIL VWF Ristocetin Cofactor Activity (RCo)", , 22. Dezember 2011 (2011-12-22), XP055027795, Gefunden im Internet: URL:http://www.kuleuven-kulak.be/irc/tromb ose/news-2/Hemosil VWF-Rco.pdf [gefunden am 2012-05-22]
- KRICKA, L.J.: "Human anti-animal antibody interferences in immunological assays", CLIN. CHEM., Bd. 45, Nr. 7, 1999, Seiten 942-956, XP003007436,
- KOHSE K P ET AL: "ANTIBODIES AS A SOURCE OF ANALYTICAL ERRORS", JOURNAL OF CLINICAL CHEMISTRY & CLINICAL BIOCHEMISTRY, BERLIN, DE, Bd. 28, Nr. 12, 1. Dezember 1990 (1990-12-01), Seiten 881-892, XP008040082,

## Beschreibung

Die vorliegende Erfindung liegt auf dem Gebiet der in vitro-Diagnostik und betrifft ein Verfahren zur Erkennung von Interferenzen in einer Probe.

Es ist bekannt, dass humanes Probenmaterial (u.a. Plasma oder Serum) anti-Immunglobulin-Antikörper gegen tierische Immunglobuline (heterophile Antikörper) und/oder gegen humane Immunglobuline (Rheumafaktor, RF) enthalten kann. Diese im Probenmaterial enthaltenen Antikörper können in vitrodiagnostische Nachweisverfahren, insbesondere Immunoassays, negativ beeinträchtigen, indem sie mit ein oder mehreren funktionellen Komponenten, die bei dem Nachweisverfahren verwendet werden (z.B. Antikörper), unspezifisch reagieren. Dadurch kommt es zu falsch zu hohen oder, seltener, zu falsch zu niedrigen Testergebnissen.

Der erste Anhaltspunkt, dass eine Probe interferierende Antikörper oder andere Störfaktoren enthalten könnte, besteht üblicherweise in einem unerwarteten Testergebnis, das gegebenfalls im Widerspruch zu anderen klinischen Befunden des Patienten stehen.

Zur Vermeidung von Interferenzen durch probenintrinsische Antikörper werden häufig sogenannte Blocking Substanzen verwendet, die die Wirkung der probenintrinsischen Antikörpern im Testansatz neutralisieren sollen. Als Blocking Substanzen werden z.B. Mischungen verschiedener polyklonaler IgG Immunglobuline, polymerisierte IgG Immunglobuline, Mischungen monoklonaler Antikörper, monoklonale anti-human IgM Immunglobuline und ähnliches verwendet (zur Übersicht siehe Kricka, L.J., Human anti-animal antibody interferences in immunological assays. Clin. Chem. 1999, 45(7): 942-956). Die Blocking Substanzen können in einem Reagenz, das für die Durchführung des Nachweisverfahrens verwendet wird, enthalten sein. Auch wenn eine Reihe kommerziell erhältlicher Blocking Substanzen zu Verfügung steht, so ist dennoch unklar, ob die Verwendung einer bestimmten Blocking Substanz, die Neutralisierung aller möglichen interferierenden Antikörper gewährleistet.

Eine Möglichkeit zu prüfen, ob eine Probe, für die ein unerwartet hohes Testergebnis in einem ersten Nachweisverfahren gemessen wurde, interferierende Substanzen enthält, besteht in der Wiederholung der Messung mit verschiedenen Verdünnungen der Probe. Es ist bekannt, dass der Störeffekt interferierender Antikörper üblicherweise nicht linear mit zunehmender Verdünnung abnimmt. Nachteilig daran ist, dass mehrere Vedünnungen gemessen werden müssen und dass interferierende Antikörper, deren Störeffekt ausnahmsweise doch linear mit zunehmender Verdünnung abnimmt, nicht erkannt werden.

Eine andere Möglichkeit zu prüfen, ob eine Probe, für die ein unerwartet hohes Testergebnis in einem ersten Sandwich-Immunoassay gemessen wurde, interferierende Substanzen enthält, besteht darin, nur einen der beiden im Sandwich-Immunoassay verwendeten Analyt-spezifischen Antikörper als Fänger- und Nachweisantikörper zu verwenden (Boscato, L.M. and Stuart, M.C., Incidence and specificity of interference in two-site immunoassays. Clin. Chem. 1986, 32(8): 1491-1495). Nachteilig daran ist, dass die Prüfung für jeden der im Immunoassay verwendeten Antikörper durchgeführt werden muss und dass dafür spezielle Reagenzien hergestellt werden müssen.

Der vorliegenden Erfindung lag die Aufgabe zugrunde, ein Verfahren zur Identifizierung einer Probe, die interferierende Störfaktoren enthält, bereit zu stellen, das weniger aufwändig ist, als die Verfahren aus dem Stand der Technik.

Die Aufgabe wird dadurch gelöst, dass eine Probe, die in einem ersten Testverfahren, bei dem die Probe zum Nachweis eines Analyten in der Probe mit ein oder mehreren funktionellen Komponenten in Kontakt gebracht wird, ein erstes Testergebnis liefert, in einem zweiten Testverfahren untersucht wird, wobei sich das zweite Testverfahren von dem ersten Testverfahren lediglich dadurch unterscheidet, dass eine funktionelle Komponente zum Nachweis des Analyten in der Probe nicht mit der Probe in Kontakt gebracht wird.

Wird in dem zweiten Testverfahren ein quantitativ signifikantes Testergebnis erhalten, zeigt dies das Vorliegen eines Störfaktors an, der mit dem ersten Testverfahren interferiert.

Das Weglassen einer funktionellen Komponente eines Testsystems bewirkt normalerweise, dass die für die Signalgenerierung erforderliche Reaktion zwischen Analyt und Bindungspartner(n) nicht stattfindet. Tritt dennoch eine Reaktion ein, die ein messbares Signal generiert, ist dieses als unspezifisch zu werten und weist auf das Vorliegen von probenintrinsischen Störfaktoren hin. Das eigentliche Testergebnis der Probe ist daher in Frage zu stellen, und der zu testende Analyt ist vorzugsweise mit Hilfe eines alternativen Verfahrens zu untersuchen.

Unter dem Begriff "interferierende Störfaktoren" sind alle probenintrinsischen Faktoren zu verstehen, die den qualitativen oder quantitativen Nachweis eines Analyten in einer Probe stören, indem sie mit einer oder mehreren Komponenten des Nachweissystems interagieren, wodurch falsch zu hohe Ergebnisse gemessen werden. Beispiele für solche Störfaktoren sind anti-Immunglobulin-Antikörper gegen tierische Immunglobuline (heterophile Antikörper), insbesondere humane anti-Maus-Antikörper (HAMA), und Antikörper gegen humane Immunglobuline (Rheumafaktor, RF).

Unter einem "ersten Testverfahren, bei dem die Probe zum Nachweis eines Analyten in der Probe mit ein oder mehreren funktionellen Komponenten in Kontakt gebracht wird" ist jedes Verfahren zum qualitativen oder quantitativen Nachweis eines Analyten in einer Probe zu verstehen. Insbesondere sind darunter Verfahren zu verstehen, bei denen die Probe mit mindestens einem analytspezifischen Bindungspartner in Kontakt gebracht wird und die Bindungsreaktion nachgewiesen wird. Beispiele sind insbesondere Immunoassays, bei denen die Antigen-Antikörper-Bindung nachgewiesen wird.

Unter einem "zweiten Testverfahren, das sich von dem ersten Testverfahren lediglich dadurch unterscheidet, dass eine funktionelle Komponente zum Nachweis des Analyten in der Probe nicht mit der Probe in Kontakt gebracht wird" ist ein Verfahren zu verstehen, das mit Ausnahme einer einzigen funktionellen Komponente alle funktionellen Komponenten und Testbedingungen des ersten Testverfahrens aufweist.

Unter einer "funktionellen Komponente zum Nachweis des Analyten" sind alle Komponenten des ersten Testverfahrens zu verstehen, die für den spezifischen Nachweis des Analyten in dem Testsystem unabdingbar sind, wie z.B. ein Analyt-bindender Bindungspartner, z.B. ein Antikörper, oder ein sekundärer Bindungspartner, der den Analyt-bindenden Bindungspartner bindet.

In einer bevorzugten Ausführungsform des erfindungsgemäßen Verfahrens handelt es sich bei dem ersten Testverfahren, bei dem die Probe zum Nachweis eines Analyten in der Probe mit ein oder mehreren funktionellen Komponenten in Kontakt gebracht wird, um einen Partikelagglutinationstest zur Bestimmung der VWF-Aktivität. In diesem VWF-Aktivitätstest wird die Probe mit isoliertem GPIbα-Protein, das in Abwesenheit von Ristocetin an VWF bindet, und mit Mikropartikeln, die mit einem monoklonalen anti-GPIbα-Antikörper beschichtet sind, als funktionellen Komponenten in Kontakt gebracht und die Agglutinationsreaktion wird gemessen (WO 2009/007051 A2).

Zur Identifizierung von interferierenden Störfaktoren in einer Probe, die mit dem vorbeschriebenen VWF-Aktivitätstest untersucht wurde, wird die Probe in einem zweiten Testverfahren untersucht, wobei sich das zweite Testverfahren von dem ersten Testverfahren lediglich dadurch unterscheidet, dass kein isoliertes GPIbα-Protein mit der Probe in Kontakt gebracht wird. Dies hat den Vorteil, dass lediglich in einem Reagenz auf eine funktionelle Komponente verzichtet wird. Alle anderen Komponenten, selbst die Pufferlösung, in dem sich die funktionelle Komponente GPIbα-Protein des ersten Testverfahrens befindet, bleiben gleich.

Alternativ kann die Probe in einem zweiten Testverfahren untersucht werden, wobei sich das zweite Testverfahren von dem ersten Testverfahren lediglich dadurch unterscheidet, dass die Mikropartikel, die mit der Probe in Kontakt gebracht werden, nicht mit anti-GPIbα-Antikörpern beschichtet sind.

In einer anderen bevorzugten Ausführungsform handelt es sich bei dem ersten Testverfahren, bei dem die Probe zum Nachweis eines Analyten in der Probe mit ein oder mehreren funktionellen Komponenten in Kontakt gebracht wird, um einen Test zur Bestimmung von Faktor XIII.

In einem beispielhaften Faktor XIII-Test wird die Probe mit Thrombin, Ca²⁺-Ionen, einem glutaminhaltigen Peptid und Glycinethylester, mit NADH (Nicotinsäureamid-Adenin-Dinukleotid-Hydrid) und mit Komponenten einer NADH-abhängigen Indikatorreaktion, nämlich mit Glutamatdehydrogenase (GLDH) und α-Ketoglutarat als funktionellen Komponenten in Kontakt gebracht und die Absorptionsänderung des Testansatzes wird gemessen (EP-A2-336353).

Zur Identifizierung von interferierenden Störfaktoren in einer Probe, die mit dem vorbeschriebenen Faktor XIII-Test untersucht wurde, wird die Probe in einem zweiten Testverfahren untersucht, wobei sich das zweite Testverfahren von dem ersten Testverfahren lediglich dadurch unterscheidet, dass kein Thrombin oder keine Ca²⁺-Ionen oder kein glutaminhaltiges Peptid oder kein Glycinethylester oder kein NADH oder keine Glutamatdehydrogenase (GLDH) oder kein α-Ketoglutarat mit der Probe in Kontakt gebracht wird.

In einer anderen bevorzugten Ausführungsform handelt es sich bei dem ersten Testverfahren, bei dem die Probe zum Nachweis eines Analyten in der Probe mit ein oder mehreren funktionellen Komponenten in Kontakt gebracht wird, um einen Test zur Bestimmung von Prothrombinfragment F1+2.

In einem beispielhaften F1+2-Test wird die Probe mit einem ersten Antikörper, der F1+2 spezifisch erkennt, und mit einem zweiten Antikörper, der den Immunkomplex aus F1+2 und dem ersten Antikörper erkennt, und mit einer ersten Komponente eines signalbildenden Systems (z.B. Chemibead), die mit dem zweiten Antikörper assoziiert ist, und mit einer zweiten Komponente eines signalbildenden Systems (z.B. Sensibead), die mit dem ersten Antikörper assoziierbar ist, als funktionellen Komponenten in Kontakt gebracht und die Chemilumineszenzentstehung im Testansatz wird gemessen (EP-A1-2168985).

Zur Identifizierung von interferierenden Störfaktoren in einer Probe, die mit dem vorbeschriebenen F1+2-Test untersucht wurde, wird die Probe in einem zweiten Testverfahren untersucht, wobei sich das zweite Testverfahren von dem ersten Testverfahren lediglich dadurch unterscheidet, dass kein F1+2-spezifischer Erstantikörper oder kein Immunkomplex-spezifischer Zweitantikörper oder keine erste oder keine zweite Komponente des signalbildenden Systems mit der Probe in Kontakt gebracht wird.

### Beispiele

### Beispiel 1: Verfahren zur Identifizierung von interferierenden Störfaktoren in einem VWF-Aktivitätstest

Bei dem INNOVANCE^{®} VWF Ac Test (Siemens Healthcare Diagnostics Products GmbH, Marburg, Deutschland) handelt es sich um einen Test zur Bestimmung der VWF-Aktivität in Plasmaproben. Zur Bestimmung der VWF-Aktivität wird die Probe zunächst mit isoliertem GPIbα-Protein, das in Abwesenheit von Ristocetin an VWF bindet, inkubiert. Anschließend werden Latexpartikel, die mit einem monoklonalen anti-GPIbα-Antikörper beschichtet sind, dem Testansatz zugegeben. Die Latexpartikel agglutinieren in Abhängigkeit von der Bindungsreaktion des GPIbα-Proteins an den VWF aus der Probe. Das isolierte GPIbα-Protein ist eine wesentliche funktionelle Komponente des Nachweisverfahrens. Ohne Zugabe von GPIbα-Protein findet keine spezifische Partikelagglutination statt.

Es wurden zwei Plasmaproben untersucht, die bekanntermaßen humane anti-Maus-Antikörper enthielten (HAMA Probe 1 und HAMA Probe 2). Als Normalprobe ohne Interferenzfaktoren wurde ein Plasmapool aus Plasmen mehrerer normaler Spender verwendet (Kontrollplasma N, Siemens Healthcare Diagnostics). Ferner wurde eine 1:3-Verdünnung von Kontrollplasma P (ein verdünnter Plasmapool aus Plasmen mehrerer normaler Spender, Siemens Healthcare Diagnostics) untersucht.

### Bestimmung der VWF-Aktivität in Anwesenheit von GPIbα (INNOVANCE^{®} VWF Ac Test)

Zur Bestimmung der VWF-Aktivität in Proben mit VWF-Aktivitäten im Normalbereich (HAMA Probe 1 und Kontrollplasma N) wurden 15 µL Probe, 30 µL Owren's Veronal Puffer, 70 µL Reaktionspuffer, 13 µL GPIbα-Reagenz (Pufferlösung enthaltend isoliertes GPIbα-Protein, das in Abwesenheit von Ristocetin an VWF bindet) und 40 µL Latexreagenz (Pufferlösung enthaltend Latexpartikel, die mit einem monoklonalen anti-GPIbα-Antikörper beschichtet sind) vermischt, und die Partikelagglutination wurde turbidimetrisch bestimmt. Mit diesem Testsetting sind VWF-Aktivitäten im Bereich von 15-150 % der Norm bestimmbar.

Zur Bestimmung der VWF-Aktivität in Proben mit geringer VWF-Aktivität (< 15 % VWF; siehe HAMA Probe 2 und verdünntes Kontrollplasma P in Tabelle 1) wurden 60 µL Probe, 70 µL Reaktionspuffer, 13 µL GPIbα-Reagenz und 40 µL Latexreagenz vermischt. Mit diesem Testsetting sind VWF-Aktivitäten im Bereich von 4-20 % der Norm bestimmbar

### Bestimmung unspezifischer Reaktionen in Abwesenheit von GPIbα

Zur Bestimmung unspezifischer Reaktionen in HAMA Probe 1 und in der Normalprobe (Kontrollplasma N) wurden 15 µL Probe, 30 µL Owren's Veronal Puffer, 70 µL Reaktionspuffer, 13 µL Pufferlösung (ohne isoliertes GPIbα-Protein, das in Abwesenheit von Ristocetin an VWF bindet) und 40 µL Latexreagenz (Pufferlösung enthaltend Latexpartikel, die mit einem monoklonalen anti-GPIbα-Antikörper beschichtet sind) vermischt, und die Partikelagglutination wurde turbidimetrisch bestimmt.

Zur Bestimmung unspezifischer Reaktionen in Proben mit geringer VWF-Aktivität (< 15 % VWF; siehe HAMA Probe 2 und verdünntes Kontrollplasma P) wurden 60 µL Probe, 70 µL Reaktionspuffer, 13 µL GPIbα-Reagenz und 40 µL Latexreagenz vermischt.

Die Ergebnisse sind in den Tabellen 1 und 2 dargestellt.

**Tabelle 1: Partikelagglutination [mE/min]**

| **Probe** | **VWF-Test mit GPIb** | **VWF-Test ohne GPIb** |
|---|---|---|
| Kontrollplasma N | 1244 | 0,2 |
| Kontrollplasma P | 251 | 1,9 |
| HAMA Probe 1 | 1524 | 930 |
| HAMA Probe 2 | 402 | 124 |

Tabelle 1 zeigt die Partikelagglutinationsraten. Während die Normalproben ohne Interferenzfaktoren (Kontrollplasma N und Kontrollplasma P) im VWF-Test ohne GPIb-Protein wie erwartet keine signifikante Reaktion zeigen, weisen die HAMA Proben im VWF-Test ohne GPIb-Zugabe unspezifische, hohe Partikelagglutinationsraten auf.

**Tabelle 2: VWF-Aktivität [% der Norm]**

| **Probe** | **VWF-Test mit GPIb** | **VWF-Test ohne GPIb** | **Differenz** | **Alternativer VWF-Test** |
|---|---|---|---|---|
| Kontrollplasma N | 93,3 | < 15 | n.a. | n.a. |
| Kontrollplasma P | 10,8 | < 4 | n.a. | n.a. |
| HAMA Probe 1 | 117,8 | 71, 9 | 45, 9 | 29, 3 |
| HAMA Probe 2 | 14,1 | 7,1 | 7,0 | 10, 6 |

Tabelle 2 zeigt die anhand der gemessenen Partikelagglutinationsraten ermittelten VWF-Aktivitäten. Während die Normalproben ohne Interferenzfaktoren (Kontrollplasma N und Kontrollplasma P) im VWF-Test ohne GPIb-Protein keine messbare VWF-Aktivität aufweisen, weisen die HAMA Proben im VWF-Test ohne GPIb-Zugabe unspezifische VWF-Aktivitäten auf.Der Vergleich mit der tatsächlichen VWF-Aktivität der HAMA Proben, die mit einem alternativen VWF-Test bestimmt wurde, zeigt, dass die im VWF-Test mit GPIb-Zugabe gemessene VWF-Aktivität ein falsch zu hohes Ergebnis darstellt.

Die Partikelagglutinationsraten "ohne GPIb" können auch benutzt werden, um mit Hilfe der Kalibrationskurve eine VWF-Aktivität zu berechnen. Im Fall der HAMA Proben lässt durch den Abzug des Wertes "ohne GPIb" von dem VWF-Wert "mit GPIb" der korrekte VWF-Gehalt der Proben ermitteln (siehe Spalte "Differenz").

## Patentansprüche

1. Verfahren zur Identifizierung von interferierenden Störfaktoren in einer Probe, die in einem ersten Testverfahren, bei dem die Probe zum Nachweis eines Analyten in der Probe mit ein oder mehreren funktionellen Komponenten zum Nachweis des Analyten in Kontakt gebracht wird, ein erstes Testergebnis liefert, **dadurch** gekennzeichnet, **dass** die Probe in einem zweiten Testverfahren untersucht wird, wobei sich das zweite Testverfahren von dem ersten Testverfahren lediglich dadurch unterscheidet, dass eine funktionelle Komponente zum Nachweis des Analyten in der Probe nicht mit der Probe in Kontakt gebracht wird.

2. Verfahren nach Anspruch 1, wobei die Probe in einem ersten Testverfahren zum Nachweis der VWF-Aktivität in der Probe mit isoliertem GPIbα-Protein und mit Mikropartikeln, die mit anti-GPIbα-Antikörpern beschichtet sind, in Kontakt gebracht wird und wobei die Probe in einem zweiten Testverfahren untersucht wird, wobei sich das zweite Testverfahren von dem ersten Testverfahren lediglich dadurch unterscheidet, dass isoliertes GPIbα-Protein nicht mit der Probe in Kontakt gebracht wird.

3. Verfahren nach Anspruch 1, wobei die Probe in einem ersten Testverfahren zum Nachweis des von Willebrand Faktors in der Probe mit isoliertem GPIbα-Protein und mit Mikropartikeln, die mit anti-GPIbα-Antikörpern beschichtet sind, in Kontakt gebracht wird und wobei die Probe in einem zweiten Testverfahren untersucht wird, wobei sich das zweite Testverfahren von dem ersten Testverfahren lediglich dadurch unterscheidet, dass die Mikropartikel, die mit der Probe in Kontakt gebracht werden, nicht mit anti-GPIbα-Antikörpern beschichtet sind.

## Claims

1. Method for identifying interfering disruptive factors in a sample which provides a first assay result in a first assay method in which the sample is contacted with one or more functional components for detecting an analyte in order to detect the analyte in the sample, **characterized in that** the sample is tested in a second assay method, wherein the second assay method differs from the first assay method merely **in that** a functional component for detecting the analyte in the sample is not contacted with the sample.

2. Method according to Claim 1, wherein the sample is contacted with isolated GPIbα protein and with microparticles coated with anti-GPIbα antibodies in a first assay method for detecting the VWF activity in the sample and wherein the sample is tested in a second assay method, wherein the second assay method differs from the first assay method merely in that isolated GPIbα protein is not contacted with the sample.

3. Method according to Claim 1, wherein the sample is contacted with isolated GPIbα protein and with microparticles coated with anti-GPIbα antibodies in a first assay method for detecting von Willebrand factor in the sample and wherein the sample is tested in a second assay method, wherein the second assay method differs from the first assay method merely in that the microparticles which are contacted with the sample are not coated with anti-GPIbα antibodies.

## Revendications

1. Procédé d'identification de facteurs perturbants d'interférence dans un échantillon, qui, dans un premier procédé de test dans lequel l'échantillon est, pour la détection d'un analyte dans l'échantillon, mis en contact avec un ou avec plusieurs constituants fonctionnels de détection de l'analyte, fournit un premier résultat de test, **caractérisé en ce que** l'on étudie l'échantillon dans un deuxième procédé de test, le deuxième procédé de test se distinguant simplement du premier procédé de test par le fait que l'on ne met pas en contact avec l'échantillon un constituant fonctionnel de détection de l'analyte.

2. Procédé suivant la revendication 1, dans lequel l'échantillon dans un premier procédé de test est, pour la détection de l'activité VWF dans l'échantillon, mis en contact avec de la protéine GPIbα isolée et avec des microparticules qui sont revêtues d'anticorps anti-GPIbα et on étudie l'échantillon dans un deuxième procédé de test, le deuxième procédé de test se distinguant simplement du premier procédé de test par le fait que l'on ne met pas de la protéine GPIbα isolée en contact avec l'échantillon.

3. Procédé suivant la revendication 1, dans lequel on met l'échantillon, dans un premier procédé de test pour la détection du facteur de Willebrand dans l'échantillon, en contact avec de la protéine GPIbα isolée et avec des microparticules qui sont revêtues d'anticorps anti-GPIbα et on étudie l'échantillon dans un deuxième procédé de test, le deuxième procédé de test se distinguant simplement du premier procédé de test par le fait que les microparticules, qui sont mises en contact avec l'échantillon, ne sont pas revêtues d'anticorps anti-GPIbα.
